# EUROPEAN PATENT APPLICATION

(11) **EP 4 814 973 A1**
(43) Date of publication of application: **30.09.2026**
(21) Application number: 23959496.3
(22) Date of filing: 24.11.2023
(51) Int. Cl.: G16C 60/00

(54) **PREDICTION DEVICE AND PREDICTION METHOD**

(71) Applicant: NGK Corporation, Nagoya-shi, Aichi 467-8530 (JP)
(72) Inventor: KONDO, Yoshio, Nagoya-shi, Aichi 467-8530 (JP); YOKOYAMA, Takashi, Nagoya-shi, Aichi 467-8530 (JP)
(74) Representative: Mewburn Ellis LLP
(86) International application number: PCT/JP2023/042195
(87) International publication number: WO 2025/109754

(57) **Abstract**

A computer acquires a parameter value set including values of a plurality of parameters each categorized into at least one of a plurality of factors affecting crystal generation. The computer performs prediction related to generation of crystal forms that a substance is able to take by inputting the parameter value set to a crystal generation model. The crystal generation model is a model that represents a crystal generation process and includes an elapsed time as an element. The computer outputs prediction result information which is information related to a result of the prediction.

## Description

### Technical Field

The present invention generally relates to a prediction technique related to a crystal form that a substance can take.

### Background Art

As a prediction technique related to a crystal form that a substance can take, a technique of calculating energy information related to a crystal structure (a structure of a crystal form) using first-principles calculation and predicting the crystal structure from the calculated energy information is known (Patent Literature 1, for example).

### Citation List

### Patent Literature

Patent Literature 1
Japanese Patent Laid-Open No. 2020-166706

### Summary of Invention

### Technical Problem

In general, first-principles calculation is calculation based on quantum mechanics (first principles), requires a large calculation load, and is used to analyze molecular behavior and intermolecular interactions on a microscopic time scale (typically on the order of several picoseconds to several tens of picoseconds). Additionally, the first-principles calculation is basically for predicting a final form of a precipitated crystal form (the process is not identified).

On the other hand, the process through which a crystal is generated from a substance (liquid phase) is a process on a macroscopic time scale.

Therefore, in order to handle a macroscopic process by using first-principles calculation, it is necessary to construct the macroscopic process by accumulating microscopic processes obtained through calculation. Consequently, the calculation load is high. Process analysis over a time scale of several minutes to several hours is practically infeasible.

### Solution to Problem

A computer acquires a parameter value set including values of a plurality of parameters each categorized into at least one of a plurality of factors affecting crystal generation. The computer performs prediction related to generation of crystal forms that a substance can take by inputting the parameter value set to a crystal generation model. The crystal generation model is a model that represents a crystal generation process and includes an elapsed time as an element. The computer outputs prediction result information which is information related to a result of the prediction.

### Advantageous Effect of Invention

According to the present invention, it is possible to perform prediction related to crystal generation on a macroscopic time scale with a lower calculation load than when using first-principles calculation.

### Brief Description of Drawings

[Figure 1] Figure 1 is a diagram illustrating a configuration of a prediction apparatus according to a first embodiment.
[Figure 2] Figure 2 is a diagram illustrating an overview of processing performed in the first embodiment.
[Figure 3] Figure 3 is a diagram illustrating an overview of precipitation prediction.
[Figure 4A] Figure 4A is a diagram illustrating that infrared rays affect precipitation of a crystal form 1.
[Figure 4B] Figure 4B is a diagram illustrating that infrared rays affect precipitation of a crystal form 2.
[Figure 5] Figure 5 is a schematic view of processing in an operation phase.
[Figure 6] Figure 6 is a diagram illustrating an example of a prediction result screen based on output information.
[Figure 7] Figure 7 is a diagram illustrating a first utilization example of the prediction apparatus.
[Figure 8] Figure 8 is a diagram illustrating a second utilization example of the prediction apparatus.
[Figure 9] Figure 9 is a diagram illustrating a configuration of a prediction apparatus according to a second embodiment.
[Figure 10] Figure 10 is a schematic view of precipitation prediction according to the second embodiment.

### Description of Embodiments

In the following description, an "interface apparatus" may mean one or more interface devices. The one or more interface devices may be at least one of the following interface devices.

### - One or more input/output (I/O) interface devices

The input/output (I/O) interface device is an interface device for at least one of an I/O device and a remote display computer. The I/O interface device for the display computer may be a communication interface device. The at least one I/O device may be either a user interface device, for example, an input device such as a keyboard and a pointing device or an output device such as a display device.

### - One or more communication interface devices

The one or more communication interface devices may be one or more communication interface devices of the same type (for example, one or more network interface cards (NICs)) or may be two or more communication interface devices of different types (for example, an NIC and a host bus adapter (HBA)).

In the following description, a "memory" means one or more memory devices, which are an example of one or more storage devices, and typically, the "memory" may be a main storage device. At least one memory device in the memory may be a volatile memory device or may be a non-volatile memory device.

In the following description, a "permanent storage apparatus" may mean one or more permanent storage devices, which are an example of the one or more storage devices. The permanent storage device may be typically a non-volatile storage device (for example, an auxiliary storage device) and may specifically be a hard disk drive (HDD), a solid state drive (SSD), a non-volatile memory express (NVME) drive, or a storage class memory (SCM), for example.

In the following description, the "storage apparatus" may mean at least the memory out of the memory and the permanent storage apparatus.

In the following description, a "processor" may mean one or more processor devices. At least one processor device may be typically a microprocessor device such as a central processing unit (CPU), or may be a processor device of another type such as a graphics processing unit (GPU). At least one processor device may be a single-core processor device or a multi-core processor device. At least one processor device may be a processor core. At least one processor device may mean a processor device in a broad sense, such as a circuit as a group of gate arrays based on a hardware description language for performing a part or entirety of processing (for example, a field-programmable gate array (FPGA), a complex programmable logic device (CPLD), or an application specific integrated circuit (ASIC)).

Although an expression like "yyy unit" may be used to explain a function in the following description, the function may be implemented by one or more computer programs being executed by the processor, may be implemented by one or more hardware circuits (for example, an FPGA or an ASIC), or may be implemented by a combination thereof. In a case where the function is implemented by the program being executed by the processor, the prescribed processing is performed by appropriately using a storage apparatus and/or an interface apparatus or the like, and the function may thus be regarded as at least a part of the processor. Any processing described with the function as the subject may be regarded as processing performed by the processor or by an apparatus including the processor. The program may be installed from a program source. The program source may be a program distribution computer or a recording medium (for example, a non-transitory recording medium) that can be read by a computer, for example. Description of each function is an example, and a plurality of functions may be combined to one function, or one function may be split into a plurality of functions.

Hereinafter, some embodiments of the present invention will be described with reference to the drawings. Note that since a substance is dissolved as a solute in a solvent and a crystal is generated through drying or the like of the solvent with the solute dissolved therein, the term "precipitation" is used as an example of the "generation" of the crystal in the following description of the embodiments.

### [First Embodiment]

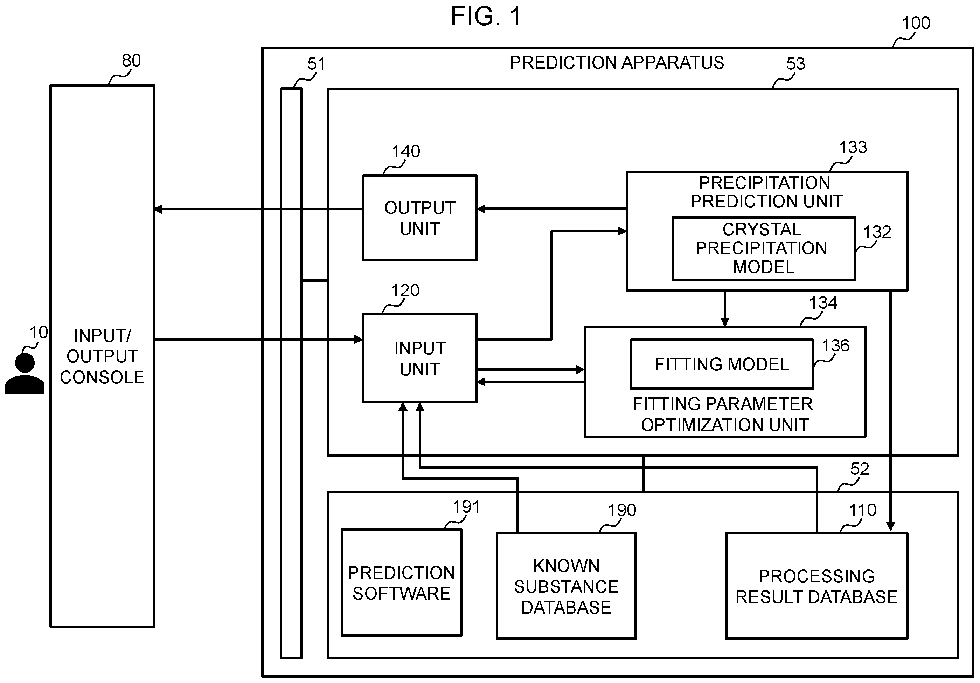

Figure 1 is a diagram illustrating a configuration of a prediction apparatus according to a first embodiment.

Although a prediction apparatus 100 is a physical computer system in the present embodiment, the prediction apparatus 100 may be a logical computer system based on a physical computer system or may be a combination of at least a part of a physical computer system and at least a part of a logical computer system. The physical computer system may be configured of one physical computer or a plurality of physical computers and includes an interface apparatus 51, a storage apparatus 52, and a processor 53 coupled thereto. The logical computer system may include a virtual machine or may include a system as a cloud computing service.

An input/output console 80 is coupled to the interface apparatus 51. The input/output console 80 is a man-machine interface apparatus to be operated by a user 10. The input/output console 80 may be, for example, an input device and a display device that the prediction apparatus 100 includes or may be a remote information processing terminal (for example, a client) communicably coupled to the prediction apparatus 100 (for example, a server). The information processing terminal may be, for example, a personal computer or a smartphone.

The storage apparatus 52 stores data and programs. The data includes, for example, a known substance database 190 including information related to each known substance and a processing result database 110 constructed or updated by processing, which will be described later. The programs include, for example, prediction software 191.

Functions such as an input unit 120, a precipitation prediction unit 133, a fitting parameter optimization unit 134, and an output unit 140 are implemented by the processor 53 executing the prediction software 191.

The input unit 120 receives inputs of information through the input/output console 80 from the user 10. The input unit 120 acquires a parameter value set from at least some of information input from the user 10, information in the known substance database 190, information in the processing result database 110, and information as results of processing performed by the fitting parameter optimization unit 134. The "parameter value set" includes a plurality of parameter values each categorized into at least one of a plurality of factors affecting precipitation of a crystal. The parameter value set includes fitting parameter values, which will be described later.

The precipitation prediction unit 133 predicts a crystal form that a substance can take, by inputting the acquired parameter value set to a crystal precipitation model 132 by the input unit 120. The crystal precipitation model 132 is a model representing a crystal precipitation process and including an elapsed time as an element. The crystal precipitation model 132 may be a machine learning model or another kind of model. In the present embodiment, at least a part of the crystal precipitation model 132 may be a model for computer simulation (for example, an approximation model), particularly, a model for a continuous simulation. The precipitation prediction unit 133 constructs or updates the processing result database 110 on the basis of a result of processing.

The fitting parameter optimization unit 134 optimizes one fitting parameter value or a plurality of fitting parameter values included in the parameter value set input to the crystal precipitation model 132. The optimization is performed using a fitting model 136, for example. The fitting model 136 may be a machine learning model or another kind of model and may be, for example, linear regression, logistic regression, a support vector machine (SVM), a decision tree model, a neural network (for example, a convolutional neural network (CNN) or a recurrent neural network (RNN)), or Bayesian optimization.

The output unit 140 generates information based on a result of prediction by the precipitation prediction unit 133 and outputs (typically displays) the information to the input/output console 80.

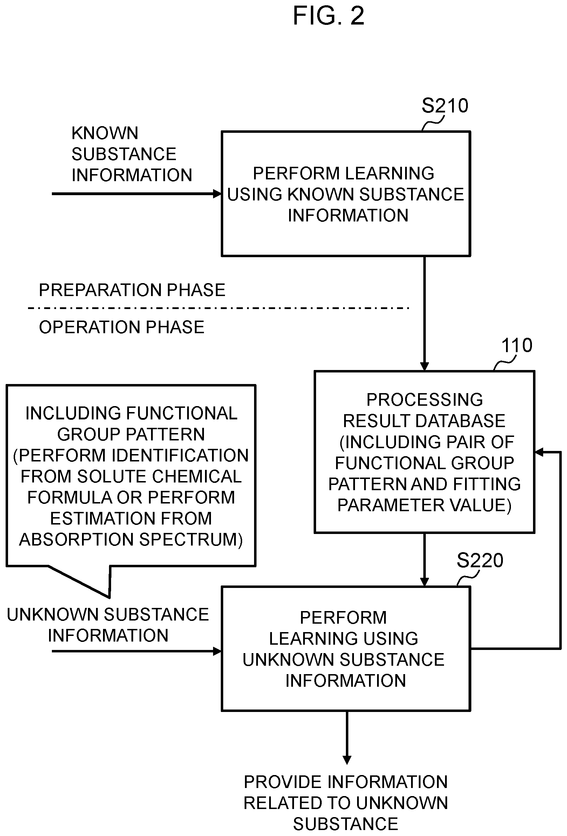

Figure 2 is a diagram illustrating an overview of processing performed in the first embodiment.

In the present embodiment, there are a preparation phase and an operation phase.

In the preparation phase, processing is performed using known substance information related to known substances (S210). In this processing, the processing result database 110 is constructed and updated. The known substance information may be information input by the user 10 or may be information acquired from the known substance database 190. The "known substances" are substances, the possible crystal forms of which have already been known. The processing result database 110 includes sets of functional group pattern and one fitting parameter value or a plurality of fitting parameter values as will be described later.

In the operation phase, processing is performed using the processing result database 110 and unknown substance information related to unknown substances (S220). The "unknown substances" are substances, the possible crystal forms of which have not yet been known. The prediction apparatus 100 can assist screening of the crystal forms that the unknown substances can take. The unknown substance information includes functional group pattern information representing one functional group pattern or a plurality of functional group patterns for each unknown substance as will be described later. The one functional group pattern or the plurality of functional group patterns may be specified from a chemical formula of the unknown substance or may be estimated from an absorption spectrum (infrared absorption spectrum) of the unknown substance.

In a case where a predetermined operation start condition is satisfied, the operation phase may be started. The operation phase may be started after the preparation phase ends, or the operation phase may be started in the middle of the preparation phase (in other words, there may be a period during which the preparation phase and the operation phase are carried out in parallel).

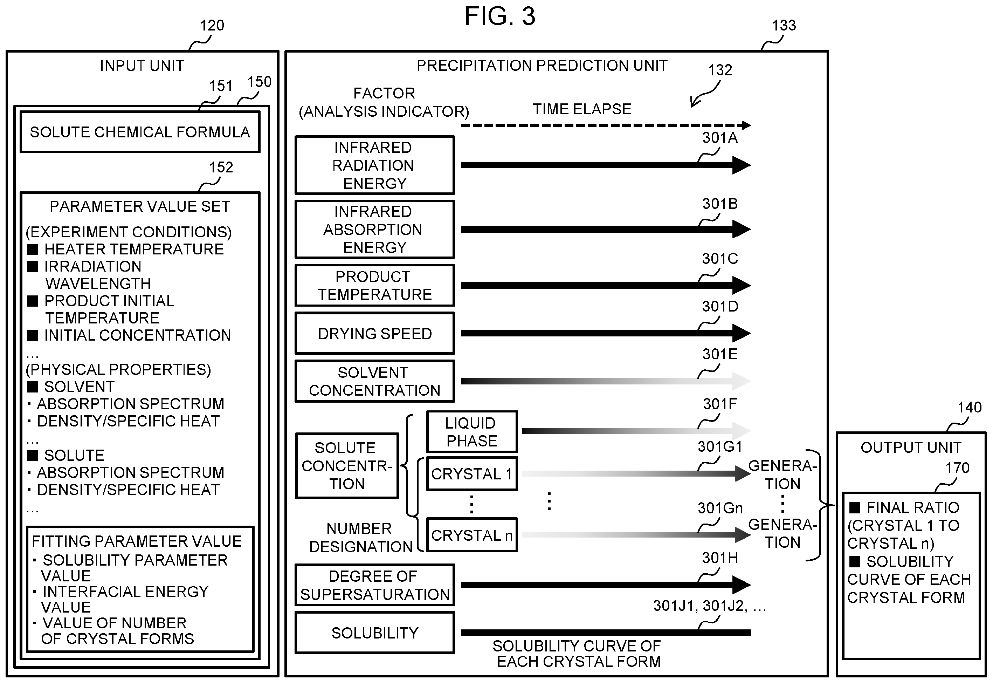

Figure 3 is a diagram illustrating an overview of precipitation prediction.

The crystal precipitation model 132 includes an equation network configured of a plurality of equations. At least a part of the equation network may be an approximation model. The equation network has one equation or a plurality of equations for each of a plurality of factors (a plurality of analysis indicators) affecting crystal precipitation. The equations are time-evolution equations (for example, differential equations) including an elapsed time as an element or non-time-evolution equations that do not include an elapsed time as an element. In Figure 3, each of equations 301A to 301H expressed by the thick straight lines with arrows is a time-evolution equation, while an equation 301J expressed by the thick straight line with no arrow is a non-time-evolution equation. The equation network may be a directed graph including equations as nodes and relationships among the equations as directed edges. Directions represented by the directed edges (the direction from start points to end points) mean that values output from equations as the start points are input to equations as the end points. The start points and the end points may be in any of the forms 1:1, many:1, 1:many, and many:many. For example, a value obtained in regard to a certain elapsed time is input and output between the equations 301.

The crystal precipitation model 132 represents a crystal precipitation process. The crystal precipitation process is a process on a macroscopic time scale, and the process corresponds to a change in thermodynamic stability. The crystal precipitation depends on a relationship between a temperature and saturation concentration for each crystal form. One exemplary relationship is a solubility curve. Therefore, the plurality of factors (the plurality of analysis indicators) include factors related to the temperature or the concentration, for example, factors such as a product temperature, a drying speed, a solvent concentration, a solute concentration, a degree of supersaturation, and solubility (note that the "product" is a solvent with the substance (solute) dissolved therein). For the solubility, there is a solubility equation 301J, which is an equation related to a solubility curve, for each crystal form. For example, there are solubility equations 301J1, 301J2, ... if there are a crystal form 1, a crystal form 2, .... Since each solubility curve is expressed by a two-dimensional orthogonal coordinate system of a temperature and a concentration and does not include an element of elapsed time, the solubility equation 301J is a non-time-evolution equation as described above.

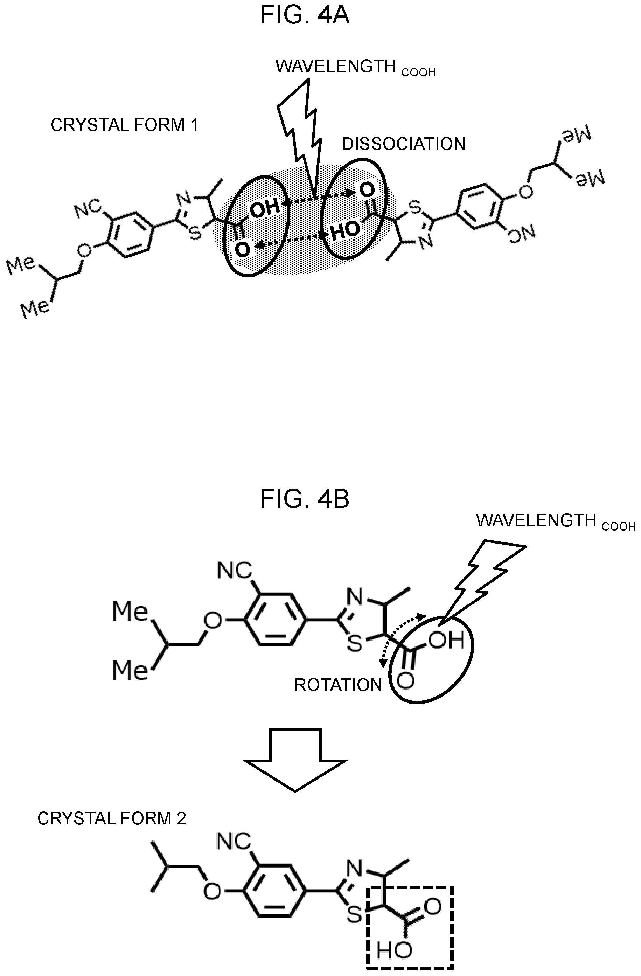

In the present embodiment, it is possible to carry out crystal precipitation under irradiation with infrared rays with a selected wavelength. In other words, it is possible to control a crystal form to be precipitated by an interaction between infrared rays with a specific wavelength and the solute (solvent). A wavelength range (a wavelength range including a peak wavelength) in which infrared absorption is significant differs depending on a functional group pattern that the substance has. Therefore, a crystal form that is likely to be precipitated and/or a crystal form that is unlikely to be precipitated is defined by infrared absorption of a wavelength depending on the functional group pattern. For example, it is assumed that the substance is Febuxostat. As illustrated in Figure 4A, a hydrogen bond between functional groups is dissociated through selective excitation of O-H stretching vibration in a carboxy group through irradiation with infrared rays with a certain wavelength W_{COOH} that is absorbed by the carboxy group, and the crystal form 1 (a crystal form in which carboxy groups of two molecules are bonded by a strong hydrogen bond) becomes unlikely to appear. On the other hand, as illustrated in Figure 4B, a carboxy group rotates and is oriented at an angle that is different from a usual angle through the irradiation with infrared rays with the same wavelength W_{COOH}, and as a result, the crystal form 2 becomes likely to appear. In this manner, it is possible to implement the crystal precipitation of causing the crystal form 1 to be unlikely to appear and causing the crystal form 2 to be likely to appear through the control of the wavelength of the infrared rays. Since the crystal precipitation is possible by such control of the wavelength of the infrared rays, there are factors related to the infrared rays, for example, analysis indicators such as infrared radiation energy and infrared absorption energy as factors. In other words, the crystal precipitation model 132 represents a crystal precipitation process under irradiation with infrared rays with a selected wavelength. Note that although both the equation 301A corresponding to the infrared radiation energy and the equation 301B corresponding to the infrared absorption energy are time-evolution equations, at least either the equation 301A or the equation 301B may be a non-time-evolution equation in a case where the energy is constant regardless of the elapsed time.

Also, there are a solvent concentration and a solute concentration as factors as described above. There is a time-evolution equation 301E for the solvent concentration. For the solute concentration, there are a time-evolution equation 301F for a liquid phase and a time-evolution equation 301G for each crystal form. Changes in darkness of the solid line arrows (straight lines) representing these time-evolution equations 301 mean concentrations with elapse of time. In other words, since the solvent concentration and the liquid phase concentration decrease with elapse of time, the solid line arrows representing the time-evolution equations 301E and 301F become gradually lighter. On the other hand, since the solute concentrations in regard to the crystal forms increase with elapse of time (since the precipitated crystals increase), the solid line arrows meaning the time-evolution equations 301G1 to 301Gn of the crystal forms 1 to n become gradually darker.

The input unit 120 acquires input information 150. The input information 150 may include a solute chemical formula 151 (an example of information representing a structure of a substance). The input information 150 includes a parameter value set 152. Parameters corresponding to parameter values included in the parameter value set 152 depend on the plurality of factors. Specifically, the parameters corresponding to the parameter values included in the parameter value set 152 include, for example, experiment condition parameters which are parameters belonging to experiment conditions (for example, a heater temperature, an irradiation wavelength (the wavelength of the infrared rays), a product initial temperature, and an initial concentration) for crystal precipitation, physical property parameters which are parameters belonging to physical properties (the solvent and/or the solute), and fitting parameters which are parameters defined to be difficult to be theoretically specified.

The parameter values in the parameter value set 152 are categorized into any of the factors and are then input to the equations 301 corresponding to the factors into which the parameter values have been categorized. Although the ratio between the parameter values and the factors (analysis indicators) may be any of 1:1, many:1, 1:many, and many:many, and a relationship between the number m of the factors and the number n of the parameter values may be any relationship, m < n in the present embodiment (that is, the number of factors is smaller than that of the parameter values).

Examples of the fitting parameters include a solubility parameter, an interfacial energy, and the number of crystal forms. The solubility parameter value is input to the solubility equation 301J. The interfacial energy value is input to a time-evolution equation 301G related to the crystal form. There are a solubility parameter value and an interfacial energy value for each of crystal forms corresponding to the value of the number of crystal forms. The value of the number of crystal forms affects the number of time-evolution equations 301G related to the crystal forms (for example, the value of the number of crystal forms is the same value as the number of time-evolution equations 301G used for the prediction). In a case where the crystal precipitation model 132 is applied to a known substance (a known compound), and the value of the number of crystal forms has already been clarified, this may not be a fitting parameter. The value of the number of crystal forms may be inferred on the basis of the functional group pattern of the substance or other information. For simple description, the fitting parameters are assumed to be a solubility parameter and interfacial energy below.

The interfacial energy is new energy generated at an interface in a case where a change in phase to a solid phase (crystal) occurs in a liquid phase, for example. In the present embodiment, the interfacial energy value is optimized as one of the fitting parameter values.

As for the solubility parameters, it is possible to state the following matters, for example. In other words, crystal precipitation depends on the temperature and the saturation concentration for each crystal form, and an exemplary relationship is a solubility curve. The solubility curve is an equation (such as a Van't Hoff equation, for example) that can originally be derived by the thermodynamics and can be uniquely determined if there is only one crystal form and heat of dissolution can be strictly measured. However, in many cases, the solubility curve for each crystal form is not accurately determined even by an experiment in practice in a case where there are multiple crystal forms. Also, examples in which the Van't Hoff equation cannot be applied to approximation are also observed. The solubility curve is defined on the basis of one or more solubility parameter value.

Hereinafter, processing in each of the preparation phase and the operation phase will be described.

Outputs obtained from the crystal precipitation model by inputting the parameter value set 152 to the crystal precipitation model 132 may include a value calculated (predicted) at each elapse point for each equation 301 and include at least one experiment condition parameter value or a plurality of experiment condition parameter values when the crystal precipitation ends. Hereinafter, the experiment condition parameter values included in the parameter value set 152 can be referred to as "first experiment condition parameter values", and the experiment condition parameter values at the time of the end of the crystal precipitation included in the outputs of the crystal precipitation model 132 can be referred to as "second experiment condition parameter values". The one first experiment condition parameter value or the plurality of first experiment condition parameter values can be referred to as a "first experiment condition parameter value set", and the one second experiment condition parameter value or the plurality of second experiment condition parameter values can be referred to as a "second experiment condition parameter value set". The first experiment condition parameter value and the second experiment condition parameter value may be present for each predetermined experiment condition item (parameter).

Processing in the preparation phase is an example of first processing. The processing in the preparation phase is performed on each of known substances that are various enough to be able to consider that many (ideally all) functional group patterns are covered.

For each of the various known substances, one or more parameter value sets 152 are prepared. Each parameter value set 152 includes one first experiment condition parameter value or a plurality of first experiment condition parameter values and one fitting parameter value or a plurality of fitting parameter values. In the present embodiment, the value of the number of crystal forms may not be a fitting parameter value for each known substance. The precipitation prediction unit 133 obtains a prediction result including the predicted one second experiment condition parameter value or the plurality of second experiment condition parameter values by inputting the parameter value set to the crystal precipitation model 132. The fitting parameter optimization unit 134 optimizes the plurality of fitting parameter values (in the present embodiment, the solubility parameter value and the interfacial energy value for each of crystal forms corresponding to the value of the number of crystal forms) on the basis of the predicted one second experiment condition parameter value or the plurality of second experiment condition parameter values. Specifically, the fitting parameter optimization unit 134 learns the one fitting parameter value or the plurality of fitting parameter values according to which the predicted one or more second experiment condition parameter values approach or overlap with the one second experiment condition parameter value or the plurality of second experiment condition parameter values obtained by the experiment (the one second experiment condition parameter value or the plurality of second experiment condition parameter values when the crystal precipitation ends in the experiment), for each parameter value set 152. More specifically, the fitting parameter optimization unit 134 learns the fitting model 136 on the basis of data including the one or more predicted second experiment condition parameter values, the one second experiment condition parameter value or the plurality of second experiment condition parameter values obtained by the experiment, and the one fitting parameter value or the plurality of fitting parameter values for each parameter value set 152, for example. Inputs to the fitting model 136 may be the predicted one or more second experiment condition parameter values, and outputs from the fitting model 136 may be the one fitting parameter value or the plurality of fitting parameter values.

The precipitation prediction unit 133 registers, in the processing result database 110, a set of data representing a functional group pattern (including at least one of the number of functional groups, the types of the functional groups, the positions of the functional groups, and a bond between the functional groups) of a substance (for example, a known substance) and a value or a value range for each fitting parameter. Depending on the functional group pattern, the set may further include content of infrared irradiation conditions (for example, one wavelength or wavelength range or a plurality of wavelengths or wavelength ranges of the infrared rays for the irradiation, an irradiation start timing and an irradiation time length for the one wavelength or wavelength range or each of the plurality of wavelengths or wavelength ranges) or other information. For example, the processing result database 110 may include, for each functional group pattern or each functional group pattern set (one functional group pattern or a plurality of functional group patterns that the known substance has), a fitting parameter value set (one fitting parameter value or a plurality of fitting parameter values (for example, a solubility parameter value and an interfacial energy value)) for each crystal form for the functional group pattern or the functional group pattern set.

Since the above processing is performed on each of known substances that are various enough to be able to assume that many functional group patterns are covered in the preparation phase, it is expected that a set including a functional group pattern and a value or a value range for each fitting parameter has been registered in the processing result database 110 for each of the many (ideally all) functional group patterns. Since the sets each including the functional group pattern and the value or the value range for each fitting parameter are accumulated in the processing result database 110 and the fitting parameter values are learned in the preparation phase in this manner, the processing in the preparation phase may be called "first learning processing".

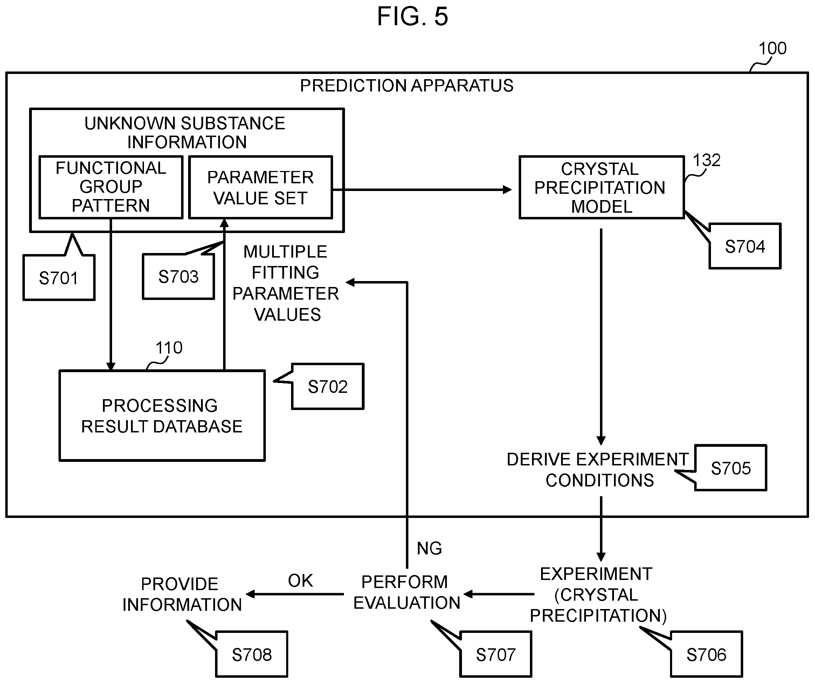

Figure 5 is a schematic diagram of processing in the operation phase.

The processing in the operation phase is an example of second processing. The processing in the operation phase is performed on unknown substances. Specifically, the following processing is performed.

(S701) The input unit 120 acquires the input information 150 related to the unknown substances and acquires unknown substance information from the input information 150. In a case where the input information 150 includes chemical formulas of the unknown substances, the input unit 120 may specify functional group patterns from the chemical formulas and acquire the unknown substance information including information representing the specified functional group patterns. In a case where the input information 150 includes information representing absorption spectra, the input unit 120 may estimate the functional group patterns of the unknown substances from the absorption spectra and the known substance database 190 (or another database representing relationships between the functional group patterns and the absorption spectra) and acquire the unknown substance information including information representing the estimated functional group patterns. The unknown substance information may include information related to one functional group pattern or a plurality of functional group patterns. Also, the unknown substance information may include parameter value sets included in the input information 150. The parameter value sets may include the first experiment condition parameter value set. In the parameter value sets, physical property parameter values may be determined on the basis of basic data of the unknown substances. The "basic data" may be data acquired by case research (comparison with similar substances, for example) of the unknown substances, analysis by an arbitrary method (for example, differential thermal analysis (DTA analysis) or a differential scanning calorimeter analysis (DSC analysis)), or the like.

(S702) The input unit 120 refers to the processing result database 110 using the one functional group pattern or the plurality of functional group patterns represented by the unknown substance information as a key.

(S703) The input unit 120 estimates a value of the number of crystal forms that an unknown substance can take, and the fitting parameter value for each fitting parameter is estimated for each of the crystal forms corresponding to the estimated value of the number of crystal forms. The value of the number of crystal forms may be estimated on the basis of the number and content of the functional group patterns represented by the unknown substance information and the processing result database 110. Also, the fitting parameter value may be estimated by either the following method (X) or (Y).

(X) the input unit 120 estimates a value range for each fitting parameter (a value range of each of one or more solubility parameters and a value range of interfacial energy). An upper limit and a lower limit of the value range for each fitting parameter may be the maximum value and the minimum value of the estimated values using the one functional group pattern or the plurality of functional group patterns as a key for the fitting parameter. The value range is a search range of the value for each fitting parameter. For example, a search range of a solubility curve (specifically, a search range of each solubility parameter value) is narrowed down in a two-dimensional orthogonal coordinate system of a temperature and a concentration. Note that the lower limit of the solubility curve may be a reference solubility curve as a solubility curve specified in crystal precipitation of the unknown substance. The "reference solubility curve" may be a curve corresponding to a crystal form that has been most often precipitated or may be an experimental-based solubility curve. The input unit 120 sets a search range (value range) for each fitting parameter for each of the crystal forms corresponding to the estimated value of the number of crystal forms (for example, includes it in the parameter value set of the unknown substance information). The fitting parameter optimization unit 134 refers to the parameter value set and searches for the fitting parameter value for each fitting parameter from the search range through machine learning (using the fitting model 136) for each crystal form. In this manner, the solubility parameter value and the interfacial energy value are defined for each of the crystal forms corresponding to the estimated number of crystal forms.

(Y) There may be a case where it is not always necessary to determine the search range of each fitting parameter, such as a case where a structure of a target unknown substance is close to structures of a plurality of known substances that are present in the information such as the known substance database 190 (for example, the distances between feature amounts of the structures are equal to or less than a predetermined value). The input unit 120 uses, as a key, structural features such as the number and content of functional group patterns represented by the unknown substance information to estimate (specify) the number of crystal forms and the fitting parameter values corresponding to structural features that are the same as or close to the corresponding structural features from the processing result database 110.

Note that the above-mentioned method (X), that is, the method of setting the search range may be utilized for prediction of known substances (for example, the processing in the preparation phase). In a case where a parameter value set including a new first experiment condition parameter value set of a certain known substance is obtained, for example, the input unit 120 may set a search range for each fitting parameter for each of crystal forms corresponding to a value of the number of crystal forms on the basis of the parameter value set, and the fitting parameter optimization unit 134 may search for a fitting parameter value corresponding to the known substance within the search range of the fitting parameter value again.

(S704) The precipitation prediction unit 133 performs prediction by inputting, to the crystal precipitation model 132, a parameter value set including the fitting parameter value set (the value of the number of crystal forms and one or more fitting parameter values for each of the crystal forms corresponding to the value of the number of crystal forms) estimated in S703. In the prediction, the one or more fitting parameter values (the solubility parameter value and the interfacial energy value) estimated in S703 for each of the crystal forms corresponding to the estimated value of the number of crystal forms are used as reference values.

(S705) The precipitation prediction unit 133 derives experiment conditions on the basis of at least a part of the prediction result in S704 and the fitting parameter values estimated in S703. Note that the experiment conditions may be arbitrarily derived by another apparatus or the user 10 instead of the prediction apparatus 100.

(S706) An experiment of precipitating a crystal from an unknown substance is performed under the experiment conditions obtained in S705.

(S707) Crystal sample evaluation (for example, XRD analysis or DSC analysis) of the crystal precipitated in S706 is performed. Note that evaluation items may include thermal analysis (for example, thermodynamic stability), crystal overview estimation by XRD analysis, and the like.

(S708) In a case where a processing end condition is satisfied in the evaluation in S707 (for example, in a case where evaluation results of all the crystal forms match the prediction), the information related to the unknown substance is provided to a utilization entity that has provided the unknown substance and will be described later.

In a case where the processing end condition is not satisfied in the evaluation in S707 (for example, in a case where an evaluation result of at least one crystal form does not match the prediction), the processing returns to S703. In S703, the fitting parameter optimization unit 134 changes (for example, makes minute correction of) the fitting parameter value estimated in S703 just previously performed. For the changing, the above-mentioned method (X) may be used. Also, the fitting model 136 may be used for the changing. For example, evaluation result values (for example, the second experiment condition parameter value set corresponding to the first experiment condition parameter value set for each first experiment condition parameter value set) and prediction result values (for example, the second experiment condition parameter value set predicted for the first experiment condition parameter value set for each first experiment condition parameter value set) for the crystal form are input to the fitting model 136, and the amount of change in parameter value of each fitting parameter for the crystal form may be based on how large the difference between the evaluation result values and the prediction result values is.

According to the processing in the operation phase, the fitting parameter values may be optimized (learned) such that the predicted second experiment condition parameter value set approaches or overlaps with the second experiment condition parameter value set obtained by the experiment in regard to each of the crystal forms corresponding to the estimated value of the number of crystal forms for the unknown substance through repetition of the processing including S703 and S704.

Note that in a case where the processing in the operation phase is ended, the precipitation prediction unit 133 may register, in the processing result database 110, a set of the one functional group pattern or the plurality of functional group patterns of the unknown substance and the estimated fitting parameter values. Also, at least a part of the information related to the unknown substance may be registered in the known substance database 190. In the operation phase, sets each including estimation (this may be referred to as optimization or learning) of the fitting parameter values and a functional group pattern and a value or a value range for each fitting parameter for the unknown substance may be accumulated in the processing result database 110, and the processing in the operation phase may thus be called "second learning processing".

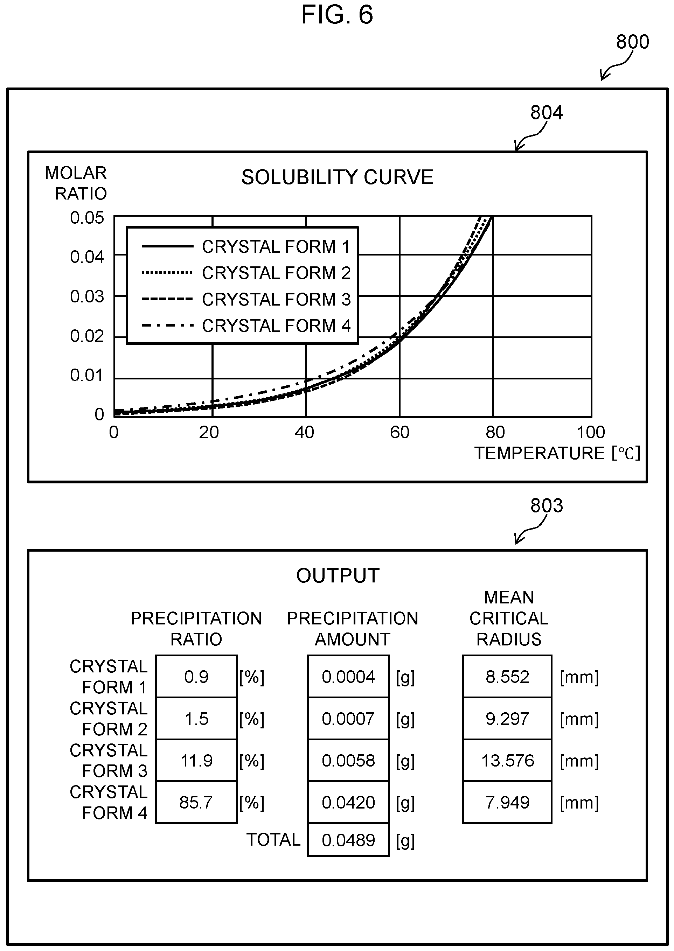

Figure 6 is a diagram illustrating an example of a prediction result screen based on output information.

In the processing in at least one of the preparation phase and the operation phase, the output unit 140 displays a prediction result screen 800 on the basis of output information 170 (see Figure 3) based on the prediction result of the precipitation prediction unit 133. The prediction result screen 800 includes, for example, crystal form information 803 and solubility information 804.

The crystal form information 803 represents crystal forms that a substance can take and a precipitation ratio for each crystal form. The crystal form information 803 may include information such as the precipitation amount and the mean critical radius for each crystal form.

The solubility information 804 includes a solubility curve (a solubility curve for each crystal form represented by the crystal form information 803) drawn in the two-dimensional orthogonal coordinate system of the temperature and the concentration. The solubility curve for each crystal form follows the solubility parameter value set (one or more solubility parameter values) estimated for the crystal form.

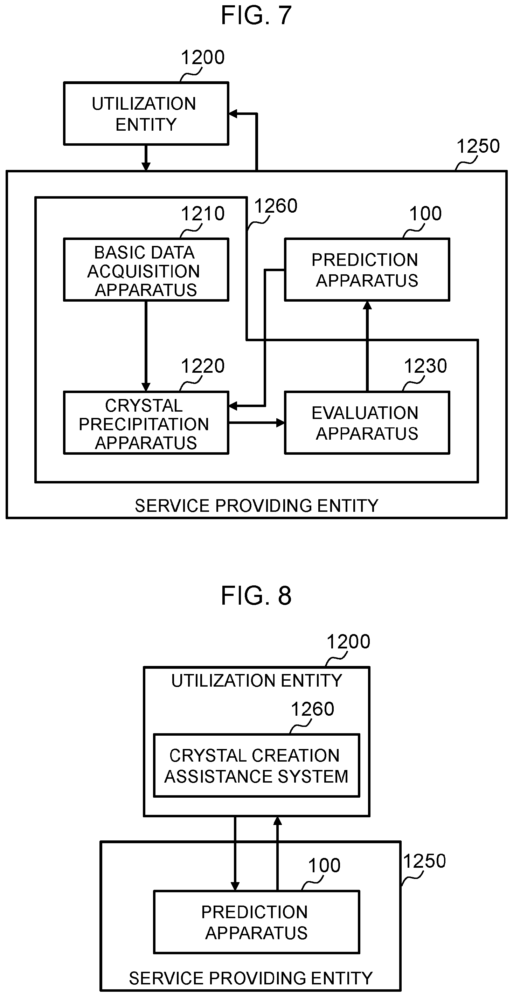

Figure 7 is a diagram illustrating a first utilization example of the prediction apparatus 100.

There are a utilization entity 1200 that provides an unknown substance as a target of prediction and a service providing entity 1250 that provides a service. In the present embodiment, the "entity" may be a natural person or a legal person and may be, for example, a company, an organization, a research institute, or another group for or not for profit.

In the first utilization example, the service providing entity 1250 includes the prediction apparatus 100 and a crystal creation assistance system 1260. The crystal creation assistance system 1260 includes at least one of a basic data acquisition apparatus 1210, a crystal precipitation apparatus 1220, and an evaluation apparatus 1230.

The basic data acquisition apparatus 1210 is an apparatus for acquiring basic data of a substance.

The crystal precipitation apparatus 1220 is an example of an experiment apparatus and is an apparatus that precipitates a crystal form. The crystal precipitation apparatus 1220 includes an infrared irradiation apparatus (for example, an infrared heater) that performs irradiation with infrared rays with a selected wavelength. The infrared irradiation apparatus evaporates and crystallizes a solvent by irradiating the solvent with a solute dissolved therein with a specific wavelength.

The evaluation apparatus 1230 is an apparatus that evaluates the crystal precipitated by the crystal precipitation apparatus 1220 (for example, an apparatus for the evaluation in S708).

For example, the following cycle is possible. In other words, the crystal precipitation apparatus 1220 precipitates a crystal of a crystal form in accordance with an experiment condition parameter value set that is the same as an experiment condition parameter value set input to the prediction apparatus 100 for each crystal form, and the evaluation apparatus 1230 evaluates the crystal. If evaluation results of all the crystal forms (for example, a second experiment condition parameter value set obtained through the experiment) are different from a prediction result (for example, a predicted second experiment condition parameter value set) of the prediction apparatus 100, then the prediction apparatus 100 performs prediction again. The prediction apparatus 100 may input a first experiment condition parameter value set of the parameter value set to the crystal precipitation apparatus 1220.

In the first utilization example, the utilization entity 1200 provides an unknown substance to the service providing entity 1250 and requests crystal form screening of the unknown substance. The service providing entity 1250 receives the request for crystal form screening, performs the crystal form screening of the unknown substance using the crystal creation assistance system 1260 and the prediction apparatus 100, and provides information as a result thereof to the utilization entity 1200.

Figure 8 is a diagram illustrating a second utilization example of the prediction apparatus.

The utilization entity 1200 includes the crystal creation assistance system 1260. The utilization entity 1200 inputs information (for example, a parameter value set) to the prediction apparatus 100 at a remote location by using, for example, the input/output console via a communication network such as the Internet, obtains output information (prediction result) from the prediction apparatus 100 in response to the input of the information, and performs crystal precipitation and evaluation using the crystal creation assistance system 1260 on the basis of the output information.

All of the components of the crystal creation assistance system 1260 may be included in either the utilization entity 1200 or the service providing entity 1250 as described above, or some of the elements in the crystal creation assistance system 1260 may be included in the utilization entity 1200 with other elements (for example, the remaining elements) of the crystal creation assistance system 1260 included in the service providing entity 1250.

Also, the above-mentioned user 10 may be a person from the utilization entity 1200, a person from the service providing entity 1250, or a person from an entity other than the entities 1200 and 1250.

### [Second Embodiment]

A second embodiment will be described. Here, differences from the first embodiment will be mainly described, and description of points common to those in the first embodiment may be omitted or simplified.

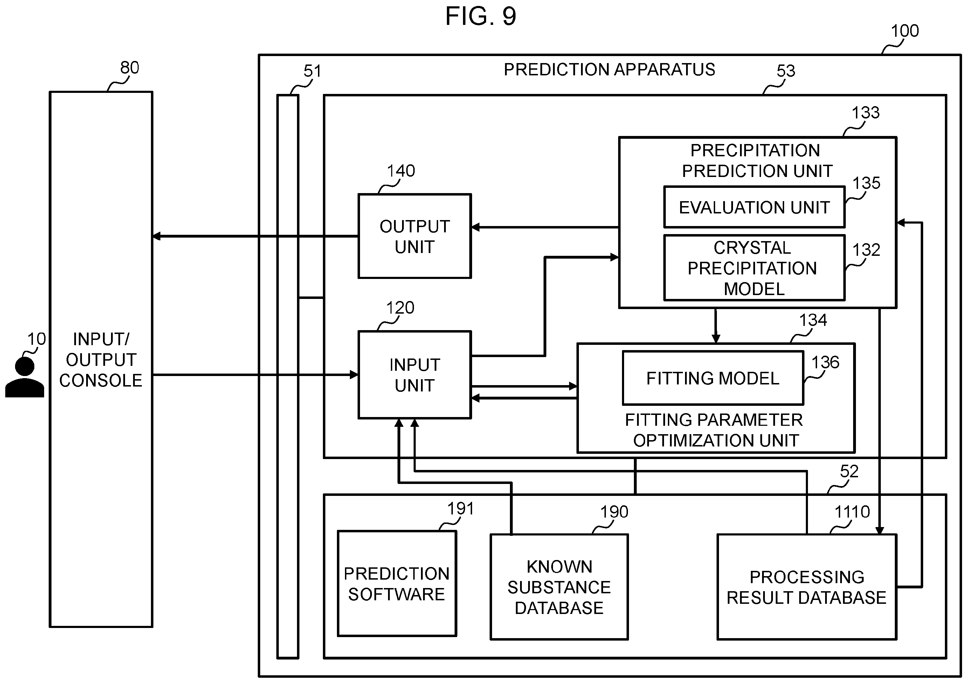

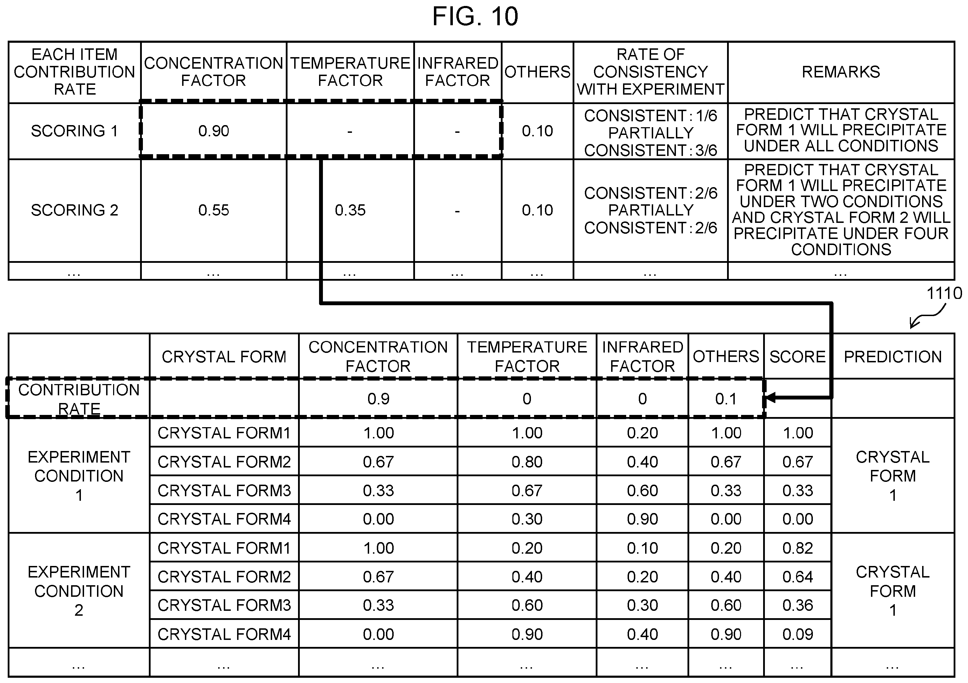

Figure 9 is a diagram illustrating a configuration of a prediction apparatus according to the second embodiment. Figure 10 is a schematic diagram of precipitation prediction according to the second embodiment.

The precipitation prediction unit 133 includes an evaluation unit 135. The evaluation unit 135 records, in a processing result database 1110, one contribution rate set or a plurality of contribution rate sets calculated in calculation using a crystal precipitation model 132. The crystal precipitation model 132 may not include an equation network as described in the first embodiment. The contribution rate set may be prepared in regard to an elapsed time for each elapsed time.

The contribution rate set is configured of contribution rates of a plurality of factors (for example, a concentration factor, a temperature factor, an infrared factor, and others) affecting crystal precipitation as illustrated in Figure 10, for example. The contribution rate for each factor is a degree of contribution of the factor to the entirety of the plurality of factors. For example, a total of the plurality of contribution rates in the contribution rate set is a predetermined value (for example, "1"). The contribution rate of each factor may differ depending on an elapsed time. According to the example illustrated in Figure 10, a certain contribution rate set is configured of a contribution rate "0.90" of a concentration factor, a contribution rate "0.00" of a temperature factor, a contribution rate "0.00" of an infrared factor, and a contribution rate "0.10" of other factors. The contribution rates may be values input and output between the factors (between the equations) (or values obtained on the basis of the values).

An influence level which is a degree of an influence of a factor on a crystal form is defined for each of the plurality of factors in regard to each crystal form that can be taken for each experiment condition (experiment condition parameter value set). A combination of the contribution rate set and a degree of factor for each experiment condition and for each factor for each crystal form is recorded in the processing result database 1110, for example. The influence level of each factor for each crystal form may be referred to as weighting of the factor for the crystal form. For example, the influence level of the concentration factor for the crystal form 1 is "1.00", and the influence level of the infrared factor for the crystal form 1 is "0.20" in regard to the experiment condition 1.

The evaluation unit 135 calculates a score for the crystal form on the basis of the influence level and the contribution rate of each factor for each crystal form in regard to each experiment condition. The precipitation prediction unit 133 predicts a crystal form to be precipitated for each experiment condition on the basis of the score for each crystal form for each experiment condition. For example, the evaluation unit 135 calculates a value (for example, a product) based on the influence level and the contribution rate for each factor for each crystal form and calculates a score (for example, a sum of a plurality of products) for the crystal form on the basis of a plurality of values calculated in regard to the plurality of factors. The evaluation unit 135 predicts that the crystal form with a high score will be precipitated. The crystal form predicted to be precipitated may be a crystal form with the highest score or may be a crystal form with a score that is equal to or greater than a predefined score. The evaluation unit 135 performs such prediction, that is, prediction of a crystal form to be precipitated for each experiment condition.

The evaluation unit 135 examines consistency (a degree of consistency) between the prediction result (predicted crystal form) and the actual experiment result under the experiment condition (the crystal form that has been precipitated under the experiment condition), that is, validity of the contribution rate set, for each experiment condition. The evaluation unit 135 calculates consistency between the prediction result and the experiment result for each contribution rate set.

The output unit 140 may display, on an input/output console 80, output information representing the contribution rate set with the most excellent consistency and the crystal form predicted by using the contribution rate set for each experiment condition.

Although some embodiments have been described hitherto, these are illustrative examples for explaining the present invention and are not intended to limit the scope of the present invention to these embodiments. The present invention can also be executed in various other forms. For example, the database in the above description is an example of data that enables an output in response to an input (for example, a key), and the data may be data with any structure (for example, this may be a structured data or unstructured data).

For example, the above description can be summarized as follows. The following summary may include supplementary explanation of the above description or explanation of modifications.

A prediction apparatus which is an example of a computer performs (A) to (C) below. (A) may be performed by the input unit 120, (B) may be performed by the precipitation prediction unit 133, and (C) may be performed by the output unit 140.
(A) Acquiring a parameter value set including values of a plurality of parameters each categorized into at least one of a plurality of factors (for example, a plurality of analysis indicators) affecting generation of a crystal.
(B) Performing prediction related to generation of crystal forms that a substance can take by inputting the acquired parameter value set to a crystal generation model which is a model that represents a crystal generation process and includes an elapsed time as an element.
(C) Outputting prediction result information which is information related to a result of the prediction.

It is thus possible to perform prediction related to crystal generation on a macroscopic time scale can be performed with a lower calculation load than when using first-principles calculation.

The parameter value set may include one first experiment condition parameter value or a plurality of first experiment condition parameter values and one fitting parameter value or a plurality of fitting parameter values. The experiment condition parameter value may be a value in regard to an experiment condition item. The one fitting parameter value or the plurality of fitting parameter values may include at least one parameter value out of a solubility parameter value (an example of a fitting parameter value related to a temperature/saturation concentration relationship) which is a parameter value related to a solubility curve, an interfacial energy value which is a value of interfacial energy, and a value of the number of crystal forms. The prediction result may include one second experiment condition parameter value or a plurality of second experiment condition parameter values (one experiment condition parameter value or a plurality of experiment condition parameter values when the crystal generation ends) obtained from the crystal generation model by inputting the parameter value set to the crystal generation model. The prediction apparatus may perform the first processing. In the first processing, the prediction apparatus (for example, the fitting parameter optimization unit 134) may learn one fitting parameter value or a plurality of fitting parameter values according to which one or more second experiment condition parameter values predicted in regard to the one first experiment condition parameter value or the plurality of first experiment condition parameter values included in the parameter value set approach or overlap with the one second experiment condition parameter value or the plurality of second experiment condition parameter values obtained by the experiment in regard to the one first experiment condition parameter value or the plurality of first experiment condition parameter values. In this manner, an improvement in prediction accuracy is expected.

Specifically, the prediction apparatus may perform target processing which is processing including (a) to (d) below, for example.
(a) The prediction apparatus (for example, the precipitation prediction unit) outputs a prediction result by inputting an acquired parameter value set to the crystal generation model. The parameter value set includes a first experiment condition parameter value set and a fitting parameter value set. The first experiment condition parameter value set includes one first experiment condition parameter value or a plurality of first experiment condition parameter values. The fitting parameter value set includes one fitting parameter value or a plurality of fitting parameter values. The prediction result includes a predicted second experiment condition parameter value set. The second experiment condition parameter value set includes one second experiment condition parameter value or a plurality of second experiment condition parameter values.
(b) The prediction apparatus (for example, the precipitation prediction unit) determines whether or not a relationship between the second experiment condition parameter value set in the prediction result in (a) and the actual (typically obtained by an experiment) second experiment condition parameter value set satisfies a predetermined condition in regard to the first experiment condition parameter value set. The expression "the relationship satisfies the predetermined condition" may mean that a difference between the second experiment condition parameter value set in the prediction result (for example, a feature amount thereof) and the actual second experiment condition parameter value set (for example, a feature amount thereof) is equal to or less than a specific amount, for example.
(c) The prediction apparatus (for example, the fitting parameter optimization unit) changes at least some fitting parameter values in the fitting parameter value set in regard to the first experiment condition parameter value set in a case where the determination result in (b) is false (that is, in a case where the difference exceeds the specific amount). The prediction apparatus (for example, the fitting parameter optimization unit) may determine the amount of change in fitting parameter value on the basis of how large the difference obtained in (b) is, and change the fitting parameter value with the determined amount of change.
(d) The prediction apparatus acquires a parameter value set including the first experiment condition parameter value set and the fitting parameter value set after the changing in (c) and performs the processing in (a) (that is, the processing returns to (a)).

Note that the prediction apparatus (for example, the precipitation prediction unit) may end the target processing in a case where the determination result in (b) is true (for example, in a case where the difference between the second experiment condition parameter value sets is equal to or less than the specific amount (for example, the difference is zero)). Also, the prediction apparatus (for example, the precipitation prediction unit) may accumulate data as a set including data representing structural features (for example, a functional group pattern) of the substance and the fitting parameter value set when the target processing of the substance is ended as a learning result in a storage apparatus. The target processing may be included in the first processing or may be included in the second processing. The substance may be a first substance (for example, a known substance) in the target processing in the first processing, and the substance may be a second substance (for example, an unknown substance) in the target processing in the second processing.

Note that in a case where the one fitting parameter value or the plurality of fitting parameter values include a solubility parameter value, it is possible to expect that an appropriate value can be estimated as a solubility parameter value which is one of parameter values that are difficult to be experimentally or theoretically obtained as appropriate values, and it is thus possible to expect an improvement in prediction accuracy. Also, in a case where the one fitting parameter value or the plurality of fitting parameter values include an interfacial energy value, it is possible to expect that an appropriate value can be estimated as an interfacial energy value which is one of parameter values that are difficult to be experimentally or theoretically obtained as appropriate values, and it is thus possible to expect an improvement in prediction accuracy. Furthermore, in a case where the one fitting parameter value or the plurality of fitting parameter values include a value of the number of crystal forms, it is possible to expect an improvement in prediction accuracy for the number of crystal forms that the substance can take.

The prediction apparatus may perform the second processing in addition to the first processing. The first processing may be processing of learning the one fitting parameter value or the plurality of fitting parameter values included in the parameter value set for a plurality of first substances. The second processing may be processing of estimating one fitting parameter value or a plurality of fitting parameter values for the second substance on the basis of the result of learning the one fitting parameter value or the plurality of fitting parameter values. One example of the first substance may be a known substance, and one example of the second substance may be an unknown substance. One example of the learning result may be the processing result database 110. At least either the first processing or the second processing may include (A) to (C). There may be a pair of the first experiment condition parameter value set and the second experiment condition parameter value set for each experiment, that is, the first experiment condition parameter value set and the second experiment condition parameter value set may have a 1:1 correspondence. Since the one fitting parameter value or the plurality of fitting parameter values for the second substance are determined using the result of learning the one fitting parameter value or the plurality of fitting parameter values, it is possible to expect that one fitting parameter value or a plurality of fitting parameter values that are appropriate can be obtained for the second substance, and it is thus possible to expect an improvement in prediction accuracy for the second substance.

One example of the crystal generation model is the crystal precipitation model 132. The crystal generation model may include a plurality of model components. The plurality of model components may be configured of a plurality of time-evolution components that correspond to some factors among a plurality of factors and each includes an elapsed time as an element and one non-time-evolution component or a plurality of non-time-evolution components that correspond to other factors among the plurality of factors and each does not include an elapsed time as an element. The one non-time-evolution component or the plurality of non-time-evolution components may include a temperature/saturation concentration relationship component, which is a model component for defining a temperature/saturation concentration relationship that is a relationship between a temperature and a saturation concentration, and which includes a fitting parameter related to the temperature/saturation concentration relationship. The plurality of time-evolution components may include model components to which a value output from the temperature/saturation concentration relationship component with the fitting parameter value related to the temperature/saturation concentration relationship having been input thereto is input. Specifically, the crystal generation model may include an equation network, the plurality of model components may be a plurality of equations constituting the equation network, and the plurality of time-evolution components may be a plurality of time-evolution equations, for example. It is possible to expect an improvement in prediction accuracy by the plurality of model components included in the crystal generation model being the one non-time-evolution component or the plurality of non-time-evolution components including the temperature/saturation concentration relationship component and the plurality of time-evolution components referring to the temperature/saturation concentration relationship component.

The result of learning the one fitting parameter value or the plurality of fitting parameter values may include a relationship between a functional group pattern and a value or a value range for the one fitting parameter or each of the plurality of fitting parameters. The functional group pattern may include at least one of the number of functional groups, the types of the functional groups, the positions of the functional groups, and the bond of the functional groups. Since the functional group pattern that a substance has affects crystal forms that the substance can take, it is possible to expect that an appropriate fitting parameter value on the basis of the above learning result is obtained and thereby it is possible to expect an improvement in prediction accuracy. Note that the "relationship" between the functional group pattern and the value or the value range of the one fitting parameter or each of the plurality of fitting parameters may be implemented as a dataset such as a database or may be implemented in another form.

Specifically, the learning result may include the fitting parameter value set (for example, the solubility parameter value and the interfacial energy value for each of the crystal forms corresponding to the value of the number of crystal forms) for functional group pattern or the functional group pattern set for each functional group pattern or for each functional group pattern set (one functional group pattern or a plurality of functional group patterns that the first substance has), for example. It is expected that the value of the number of crystal forms and the solubility parameter value and the interfacial energy value for each of the crystal forms corresponding to the value of the number of crystal forms are estimated on the basis of the one functional group pattern or the plurality of functional group patterns for the second substance and the learning result.

The crystal generation model may include association with a functional group affecting element (an element affecting the functional groups). The functional group affecting element is an element that causes a hydrogen bond between the functional groups, dissociation of the hydrogen bond between the functional groups, and the like, and a specific example thereof is infrared rays as described above. Wavelength ranges where infrared absorption is significant differ depending on the functional group pattern, and it is thus expected that more crystal forms are generated by controlling the wavelength of the infrared rays for irradiation. In other words, it may be possible to recognize crystal forms that have not been covered until now, and it is possible to expect that the possibility of missing of predicted crystal forms is reduced since the crystal generation model expresses the crystal generation process using the functional group affecting element such as the infrared rays.

In the second processing, the prediction apparatus may estimate the value of the number of crystal forms that the second substance can take on the basis of the learning result (for example, accumulated learning results) and the one functional group pattern or the plurality of functional group patterns for the second substance. The prediction apparatus may learn the one fitting parameter value or the plurality of fitting parameter values according to which the one second experiment condition parameter value or the plurality of second experiment condition parameter values predicted for the second substance approach or overlap with the one second experiment condition parameter value or the plurality of second experiment condition parameter values obtained by the experiment for the second substance, for each of the crystal forms corresponding to the number estimated for the second substance. In this manner, optimization of the fitting parameter values for the second substance is expected.

In the second processing, the prediction apparatus may reflect, to the learning result, a relationship between the one functional group pattern or the plurality of functional group patterns and the value or the value range searched for the one fitting parameter or each of the plurality of fitting parameters for the second substance. In this manner, the learning result becomes further sufficient, and an improvement in precision of the prediction result in the following second processing is expected.

In the first processing and/or the second processing, the prediction apparatus may estimate the search range for the one fitting parameter or each of the plurality of fitting parameters in addition to the number of crystal forms that the substance can take on the basis of the learning result and the one functional group pattern or the plurality of functional group patterns of the substance, and may search for the fitting parameter value from the estimated search range for the one fitting parameter or each of the plurality of fitting parameters for each of the crystal forms corresponding to the number estimated for the substance. Since the search range is narrowed in this manner, it is possible to expect that the calculation load related to the searching for the fitting parameter value is reduced.

The prediction result information may include at least either information representing a ratio of each of the crystal forms that the substance can take or information representing a temperature/saturation concentration relationship for each of the crystal forms that the substance can take. This facilitates recognition of the prediction result through display of the prediction result information.

In (B), the prediction apparatus may perform the following processing. In this manner, an improvement in prediction accuracy is expected.
- To set one contribution rate set or a plurality of contribution rate sets configured of contribution rates of the respective factors (for each of factors defined in regard to the crystal generation model, for example).
- To calculate a score for each crystal form for each experiment condition on the basis of the influence level of each factor defined for each of the crystal forms that can be taken for each experiment condition (experiment condition parameter value set) for crystal generation and a contribution rate of each of the factors in the contribution rate set at each elapsed time.
- To predict the crystal form to be precipitated for each experiment condition on the basis of the score for each crystal form under each experiment condition for each contribution rate set.
- To calculate consistency between the prediction result using the contribution rate set under each experiment condition and the actual experiment result under each experiment condition for each contribution rate set.

There may be an experiment condition parameter value at each of a plurality of points for each experiment condition parameter (for example, each factor (analysis indicator)). For example, a first experiment condition parameter value may be an example of the experiment condition parameter value at a first point. A second experiment condition parameter value may be an example of the experiment condition parameter value at a second point. The "plurality of points" described here may include two or more points in the elapse of time during calculation (for example, simulation) using the crystal generation model or may include two or more points in an actual elapsed time (for example, an elapsed time in the actual experiment). An example of the second point may be a point of crystal precipitation. An example of the experiment condition parameter value at the first point may be an experiment condition parameter value to be input to the crystal generation model, for example, the first experiment condition parameter value. An example of the experiment condition parameter value at the second point may be an experiment condition parameter value to be output from the crystal generation model, for example, the second experiment condition parameter value. A fitting parameter value according to which the one experiment condition parameter value or the plurality of experiment condition parameter values predicted at the second point approach or overlap with the one actual (obtained in the experiment, for example) experiment condition parameter value or the plurality of actual experiment condition parameter values at the second point may be learned. For example, a fitting parameter value according to which the feature amount of the one experiment condition parameter value or the plurality of experiment condition parameter values predicted at the second point approaches or overlaps with the feature amount of the one actual experiment condition parameter value or the plurality of actual experiment condition parameter values at the second point may be learned.

According to the above description, it is possible to express the method of optimizing the one fitting parameter value or the plurality of fitting parameter values as follows, for example. Note that "one fitting parameter value or a plurality of fitting parameter values" in the following expression may include at least one of the solubility parameter value, the interfacial energy value, and the value of the number of crystal forms similar to the above description. The one fitting parameter value or the plurality of fitting parameter values may include the solubility parameter value and the interfacial energy value for each of the crystal forms corresponding to the value of the number of crystal forms. Also, one example of the following "prediction model" may be the crystal generation model. The prediction model may be a model including an elapsed time as an element. At least a part of the prediction model may be an approximation model. Furthermore, a model for fitting parameter values (for example, a model of the same type as the fitting model 136) may be used for learning the fitting parameter values.

### <Expression Example of Method of Optimizing Fitting Parameter Values>

A method of optimizing one fitting parameter value or a plurality of fitting parameter values affecting a prediction result using a prediction model in a parameter value set to be input to the prediction model, the method including:
learning the one fitting parameter value or the plurality of fitting parameter values according to which one parameter value or a plurality of parameter values included in the prediction result using the prediction model approach or overlap with one parameter value or a plurality of parameter values as actual values, for one predetermined parameter item or a plurality of predetermined parameter items.

### Reference Signs List

100 Prediction apparatus

## Claims

1. A prediction method performed by a computer comprising:
(A) acquiring a parameter value set including values of a plurality of parameters each categorized into at least one of a plurality of factors affecting crystal generation;
(B) performing prediction related to generation of crystal forms that a substance is able to take by inputting the parameter value set to a crystal generation model which is a model that represents a crystal generation process and includes an elapsed time as an element; and
(C) outputting prediction result information which is information related to a result of the prediction.

2. The prediction method according to claim 1, wherein
the parameter value set includes one first experiment condition parameter value or a plurality of first experiment condition parameter values and one fitting parameter value or a plurality of fitting parameter values,
the experiment condition parameter values are values related to experiment condition items,
the one fitting parameter value or the plurality of fitting parameter values include at least one parameter value out of a solubility parameter value which is a parameter value related to a solubility curve, an interfacial energy value which is a value of interfacial energy, and a value of the number of crystal forms,
the result of the prediction includes one second experiment condition parameter value or a plurality of second experiment condition parameter values which are one experiment condition parameter value or a plurality of experiment condition parameter values when crystal generation ends obtained from the crystal generation model by inputting the parameter value set to the crystal generation model, and
the computer performs first processing which is processing of learning the one fitting parameter value or the plurality of fitting parameter values according to which one or more second experiment condition parameter values predicted for one first experiment condition parameter value or a plurality of first experiment condition parameter values included in the parameter value set approach or overlap with one second experiment condition parameter value or a plurality of second experiment condition parameter values obtained by an experiment for the one first experiment condition parameter value or the plurality of first experiment condition parameter values.

3. The prediction method according to claim 2, wherein
the first processing is processing of learning one fitting parameter value or a plurality of fitting parameter values included in the parameter value set for a plurality of first substances,
the computer performs second processing which is processing of estimating one fitting parameter value or a plurality of fitting parameter values for a second substance on the basis of a result of learning the one fitting parameter value or the plurality of fitting parameter values, and
at least either the first processing or the second processing includes (A) to (C).

4. The prediction method according to claim 2, wherein
the crystal generation model includes a plurality of model components,
the plurality of model components are configured of
a plurality of time-evolution components that correspond to some factors among the plurality of factors and each includes an elapsed time as an element, and
one non-time-evolution component or a plurality of non-time-evolution components that correspond to other factors among the plurality of factors and each does not include an elapsed time as an element,
the one non-time-evolution component or the plurality of non-time-evolution components include a temperature/saturation concentration relationship component, which is a model component for defining a temperature/saturation concentration relationship that is a relationship between a temperature and a saturation concentration, and which includes a fitting parameter related to the temperature/saturation concentration relationship, and
the plurality of time-evolution components include a model component to which a value output from the temperature/saturation concentration relationship component with the fitting parameter value related to the temperature/saturation concentration relationship having been input thereto is input.

5. The prediction method according to claim 4, wherein
the crystal generation model includes an equation network,
the plurality of model components are a plurality of equations constituting the equation network, and
the plurality of time-evolution components are a plurality of time-evolution equations.

6. The prediction method according to claim 3, wherein
the result of learning the one fitting parameter value or the plurality of fitting parameter values includes a relationship between a functional group pattern and a value or a value range for the one fitting parameter or each of the plurality of fitting parameters, and
the functional group pattern includes at least one of the number of functional groups, types of the functional groups, positions of the functional groups, and a bond between the functional groups.

7. The prediction method according to claim 6, wherein the crystal generation model includes association with a functional group affecting element which is an element affecting the functional groups.

8. The prediction method according to claim 7, wherein the functional group affecting element is infrared rays.

9. The prediction method according to claim 6, wherein
in the second processing,
a value of the number of crystal forms that the second substance is able to take on the basis of the result of learning and one functional group pattern or a plurality of functional group patterns of the second substance is estimated, and
one fitting parameter value or a plurality of fitting parameter values according to which one second experiment condition parameter value or a plurality of second experiment condition parameter values predicted for the second substance approach or overlap with one second experiment condition parameter value or a plurality of second experiment condition parameter values obtained by an experiment for the second substance are learned for each of the crystal forms of the number estimated for the second substance.

10. The prediction method according to claim 9, wherein in the second processing, a relationship between one functional group pattern or a plurality of functional group patterns and a value or a value range searched for the one fitting parameter or each of the plurality of fitting parameters is reflected to the result of learning for the second substance.

11. The prediction method according to claim 6, wherein
in the first processing and/or the second processing,
a search range for the one fitting parameter value or each of the plurality of fitting parameter values is estimated in addition to the number of crystal forms that the substance is able to take, on the basis of the result of learning and one functional group pattern or a plurality of functional group patterns for the substance, and
a fitting parameter value is searched for from the estimated search range in relation to the one fitting parameter value or each of the plurality of fitting parameter values for each of the crystal forms for the substance.

12. The prediction method according to claim 1, wherein the prediction result information includes at least either information representing a ratio of each of crystal forms that the substance is able to take or information representing a temperature/saturation concentration relationship which is a relationship between a temperature and a saturation concentration for each of the crystal forms that the substance is able to take.

13. The prediction method according to claim 1, wherein
in (B),
one contribution rate set or a plurality of contribution rate sets configured by contribution rates for factors defined in relation to the crystal generation model are set,
a score for each crystal form is calculated for each experiment condition parameter value set on the basis of an influence level for each factor defined for each crystal form that is able to be taken for each experiment condition parameter value set which includes one or more experiment condition parameter values for crystal generation and a contribution rate of each factor in the contribution rate set at each elapsed time,
a crystal form to be precipitated is predicted for each experiment condition parameter value set on the basis of the score for each crystal form for each experiment condition parameter value set for each contribution rate set, and
consistency between a prediction result using the contribution rate set for each experiment condition parameter value set and an actual experiment result for each experiment condition parameter value set is calculated for each contribution rate set.

14. A prediction apparatus comprising:
an input unit that acquires a parameter value set including values of a plurality of parameters each categorized into at least one of a plurality of factors affecting crystal generation;
a prediction unit that performs prediction related to generation of crystal forms that a substance is able to take by inputting the parameter value set to a crystal generation model which is a model that represents a crystal generation process and includes an elapsed time as an element; and
an output unit that outputs prediction result information which is information related to a result of the prediction.

15. A recording medium having recorded thereon a computer program for causing a computer to execute:
(A) acquiring a parameter value set including values of a plurality of parameters each categorized into at least one of a plurality of factors affecting crystal generation;
(B) performing prediction related to generation of crystal forms that a substance is able to take by inputting the parameter value set to a crystal generation model which is a model that represents a crystal generation process and includes an elapsed time as an element; and
(C) outputting prediction result information which is information related to a result of the prediction.

16. A computer program that causes a computer to execute:
(A) acquiring a parameter value set including values of a plurality of parameters each categorized into at least one of a plurality of factors affecting crystal generation;
(B) performing prediction related to generation of crystal forms that a substance is able to take by inputting the parameter value set to a crystal generation model which is a model that represents a crystal generation process and includes an elapsed time as an element; and
(C) outputting prediction result information which is information related to a result of the prediction.
